# EUROPÄISCHE PATENTSCHRIFT

(11) **EP 2 563 444 B1**
(45) Veröffentlichungstag und Bekanntmachung des Hinweises auf die Patenterteilung: **04.09.2019**
(21) Anmeldenummer: 10800899.6
(22) Anmeldetag: 30.11.2010
(51) Int. Cl.: A61M 16/01

(54) **VORRICHTUNG ZUR APPLIKATION MINDESTENS EINES MEDIZINISCHEN GASES AN EINEN MIT HILFE EINES ANÄSTHESIEGERÄTS BEATMETEN PATIENTEN**
DEVICE FOR SUPPLYING AT LEAST ONE MEDICAL GAS TO A PATIENT RECEIVING ARTIFICIAL RESPIRATION WITH THE AID OF AN ANESTHESIA MACHINE
DISPOSITIF POUR ADMINISTRER AU MOINS UN GAZ MÉDICAL À UN PATIENT SOUS ASSISTANCE RESPIRATOIRE AU MOYEN D'UN APPAREIL D'ANESTHÉSIE

(30) Priorität: 29.04.2010 DE 102010016699
(43) Veröffentlichungstag der Anmeldung: 06.03.2013
(73) Patentinhaber: Joost, Thilo, 61169 Friedberg (DE)
(72) Erfinder: KÖBRICH, Rainer, 68809 Neulußheim (DE); JOOST, Thilo, 61169 Friedberg (DE)
(74) Vertreter: Sattler de Sousa e Brito, Clara
(86) Internationale Anmeldenummer: PCT/EP2010/068560
(87) Internationale Veröffentlichungsnummer: WO 2011/134548

(56) Entgegenhaltungen:
- WO-A1-2009/113899
- DE-A1- 3 416 291
- FR-A1- 2 911 281
- US-A- 4 686 974
- US-A- 5 520 172
- US-A- 5 845 633
- US-A- 6 032 665
- US-A1- 2005 039 740
- US-A1- 2007 062 527

## Beschreibung

Die Erfindung betrifft eine Vorrichtung zur Applikation mindestens eines medizinischen Gases an einen beatmeten und anästhesierten Patienten. Ein Anästhesiegerät erzeugt in einer Atemgas zuführenden ersten Leitung einen Atemgasfluss. Dem Atemgasfluss wird eine vorbestimmte Menge eines zu applizierenden medizinischen Gases zugesetzt. Über eine zweite Leitung werden zumindest das vom Patienten ausgeatmete Gas und der Anteil des vom Anästhesiegerät in die erste Leitung eingebrachte Atemgas sowie das in eine Patientenzuleitung eingeleitete medizinische Gas, die vom Patienten nicht eingeatmet worden sind, abgeführt.

Aus den Dokumenten EP 0 937 479 B1, EP 0 937 479 B1, US 5,558,083, EP 0 786 264 B1, EP 1 516 639 B1, und EP 0 723 466 B1 sind Vorrichtungen und Verfahren zum Verabreichen von Stickstoffmonoxid mit kontinuierlichen und gepulsten Zeitverläufen an einen beatmeten Patienten bekannt. Dabei sind Regelventile zum Einstellen der Stickstoffmonoxidmenge vorgesehen, die jedoch jeweils bauartbedingt unter konkreten gegebenen Druckverhältnissen eine definierte Gasmenge pro Zeiteinheit durchlassen. Somit ist man darauf angewiesen, dass das medizinische Gas in einer für die Vorrichtung geeigneten Konstellation bereitgestellt wird und dem Patienten im geöffneten Zustand der Regulierungsmittel eine zur Behandlung geeignete Gasmenge zur Verfügung gestellt wird. Jedoch ist es wünschenswert, den beatmeten Patienten erforderlichenfalls von den Wirkstoffen des medizinischen Gases kontinuierlich oder schrittweise zu entwöhnen und die Gasmenge des applizierten medizinischen Gases pro Zeiteinheit zu verringern. Bei hohen durch die Gasquelle bereitgestellten Konzentrationen des medizinischen Gases und bei einer sehr geringen zuzuführenden Gasmenge ist somit eine genaue Dosierung geringer Gasmengen erforderlich, wohingegen bei Gasquellen mit geringer Konzentration des medizinischen Gases und Applikation relativ großer Mengen des medizinischen Gases mit Hilfe der Regulierungsmittel wesentlich größere Gasmengen in die erste Leitung eingebracht werden müssen.

FR2911281A1 offenbart eine Vorrichtung zur Applikation von NO an einen Patienten mit einer vom Beatmungsgerät zuführenden ersten Leitung und einer vom Patienten ausgeatmetes Gas abführenden zweiten Leitung, mit einer Patientenzuleitung, wobei ein erstes Ende der ersten Leitung, ein erstes Ende der zweiten Leitung und ein erstes Ende der Patientenzuleitung über ein Verbindungsstück miteinander verbunden sind, mit einem einen Atemgasfluss erzeugenden Beatmungsgerät, mit Zuführmitteln zum Zuführen von NO in die Patientenleitung, mit zwei parallel angeordneten Regulierungsmittel, die eine NO-Gasquelle und die Patientenleitung verbinden, wobei jedes Regulierungsmittel im geöffneten Zustand eine Verbindung zwischen der Gasquelle und der Patientenleitung herstellt, mit einer Steuereinheit, die die Regulierungsmittel derart ansteuert, dass sie nacheinander mehrere Gasimpulse von NO in die Patientenleitung einleiten, wobei die Steuereinheit den zeitlichen Beginn der Einatemphasen ermittelt, und die Steuereinheit die Regulierungsmittel derart ansteuert, dass diese am ermittelten Beginn der Einatemphase einen Gasimpuls in die Patientenzuleitung einleiten.

US5520172 offenbart einen Anästhesiegerät das einen Atemgasfluss erzeugt, mit einer Atemgas vom Anästhesiegerät zuführenden ersten Leitung und einer vom Patienten ausgeatmetes Gas abführenden zweiten Leitung, mit einer Patientenzuleitung, wobei ein erstes Ende der ersten Leitung, ein erstes Ende der zweiten Leitung und ein erstes Ende der Patientenzuleitung über ein Verbindungsstück miteinander verbunden sind, wobei ein zweites Ende der ersten Leitung und ein zweites Ende der zweiten Leitung miteinander verbunden sind, so dass ein geschlossener Kreislauf ausgebildet ist.

Aufgabe der Erfindung ist es, eine Vorrichtung zur Applikation mindestens eines medizinischen Gases an einem mit Hilfe eines Anästhesiegeräts beatmeten und anästhesierten Patienten anzugeben, bei der die zu applizierende Gasmenge einfach einstellbar ist.

Diese Aufgabe wird durch eine Vorrichtung mit den Merkmalen des unabhängigen Vorrichtungsanspruchs 1 gelöst. Vorteilhafte Weiterbildung der Erfindung ist in dem abhängigen Patentanspruch angegeben, Weiterhin wird auch ein entsprechendes Verfahren offenbart, das jedoch zur Erfindung nicht gehört.

Durch das Verfahren sowie die Vorrichtung zur Applikation mindestens eines medizinischen Gases an einem mit Hilfe eines Anästhesiegeräts beatmeten und anästhesierten Patienten wird erreicht, dass über die beiden parallel angeordneten Regulierungsmittel jeweils das medizinische Gas in den dort vom Anästhesiegerät erzeugten Atemgasfluss in der ersten Leitung eingebracht und so dem Patienten zugeführt werden kann. Durch die Öffnung eines der Regulierungsmittel oder beider Regulierungsmittel zum Erzeugen der Gasimpulse kann die Menge, insbesondere das Volumen des in die Patientenzuleitung eingebrachten medizinischen Gases bei einer entsprechenden Wahl der Impulslänge und Impulsfolge eingestellt werden. Dabei wird zunächst der zeitliche Beginn mindestens einer Einatemphase des Patienten ermittelt. Anschließend werden mit Hilfe einer Steuereinheit die Regulierungsmittel derart angesteuert, dass am ermittelten Beginn der Einatemphase ein Gasimpuls in die Patientenzuleitung eingeleitet wird. Ein zweites Ende der ersten Leistung und eine zweites Ende der zweiten Leitung sind miteinander verbunden, so dass ein geschlossener Kreislauf ausgebildet ist. Somit wird verhindert, dass zur Anästhesie des Patienten benutztes Anästhesiemittel in die Umgebungsluft, insbesondere in einen Operationssaal, gelangt.

Unter dem Einleiten am Beginn der Einatemphase kann das Einleiten genau am zeitlich Anfang der Einatemphase oder mit einem voreingestellten zeitlichen Abstand nach dem Anfang der Einamtemphase verstanden werden. Der zeitliche Anstand ist hierbei insbesondere derart gewählt, dass der Gasimpulse während der Einatemphase, vorzugsweise im ersten Drittel der Einatemphase, eingeleitet wird.

Vorzugsweise wird während der Einatemphase nur dieser eine Gasimpuls eingeleitet. Während der sich an die Einatemphase anschließenden Atemstillstandsphase und der sich hieran anschließenden Ausatemphase wird insbesondere kein medizinisches Gas eingeleitet. Hierdurch wird erreicht, dass das gesamte während der Einatemphase vom Patienten aufzunehmende Gas bereits zu Beginn der Einatemphase dem Atemgas zugeführt wird und von dem Patienten aufgenommen werden kann. Somit erfolgt zum Einen keine Verschwendung von medizinischem Gas während der Atemstillstandsphase und/oder der Ausatemphase und zum Anderen kann das medizinische Gas somit tief in die Lunge gelangen und dadurch besser aufgenommen werden. Ferner wird hierdurch erreicht, dass bei entsprechender Dosierung der Menge des medizinischen Gases dieses vollständig oder zumindest zum Großteil vom Patienten aufgenommen wird, so dass es zu keiner Kumulation des medizinischen Gases in dem geschlossenen Kreislauf kommt. Somit kann die therapeutisch optimale Menge an medizinischem Gas dem Patienten verabreicht werden und es werden Reaktionen des medizinischen Gases mit anderen Bestandteilen des in dem Kreislauf befindlichen Gasgemisches vermieden oder zumindest reduziert.

Das medizinische Gas wird insbesondere stoßartig zu Beginn der Einatemphase zugeführt, damit von Beginn der Einatemphase an möglich viel medizinisches Gas durch den Patienten ausgenommen werden kann und der Patient das medizinische Gas vollständig oder zumindest zum Großteil aufnimmt. Unter dem stoßartigen Zuführen wird insbesondere verstanden, dass die zuzuführende Menge an medizinischem Gas innerhalb möglichst kurzer Zeit mit einem großen Volumenstrom zugeführt wird.

Durch die geeignete Auswahl der Dimensionierung der Regulierungsmittel können auf diese Weise auch Gasquellen mit unterschiedlicher Konzentration des medizinischen Gases eingesetzt werden, ohne dass bauliche Veränderungen der Vorrichtung zur Applikation des medizinischen Gases erforderlich sind. Somit bieten sowohl das Verfahren als auch die Vorrichtung eine variable Einstellung der zu applizierenden Gasmenge in großen Verstellbereichen und somit ein breites Konzentrationsspektrum. Der durch die Gasimpulse bewirkte impulsförmige Partialdruck ist bis in die Atemwege des beatmeten Patienten messbar.

Wie bereits erwähnt, führt die durch die Gasimpulse bewirkte Gaseinmischung sehr schnell zu einer homogenen Partialdrucksituation.

Ferner ist es vorteilhaft, wenn die Regulierungsmittel mit Hilfe der Steuereinheit derart angesteuert werden, dass eine zu dem Gasimpuls festgelegte Gasmenge und/oder zu dem Gasimpuls festgelegtes Gasvolumen in die Patientenzuleitung eingeleitet wird. Dadurch lässt sich die für die Applikation erforderliche Gasmenge bzw. das erforderliche Gasvolumen auf einfache Art und Weise in die Patientenzuleitung einbringen.

Besonders vorteilhaft ist es, wenn das medizinische Gas NO (Stickstoffmonoxid) enthält. Das medizinische Gas kann insbesondere als Gasgemisch aus NO (Stickstoffmonoxid) und N₂ (Stickstoff) bereitgestellt werden. Besonders vorteilhaft hat sich auch ein Gasgemisch aus NO (Stickstoffmonoxid) und He (Helium) erwiesen, da insbesondere durch Helium besonders kurze Reaktions- und Ansprechzeiten erreicht werden. Dadurch kann insbesondere bei Neugeborenen sowie bei Frühgeborenen und den von diesen Patienten relativ geringen eingeatmeten Mengen des Gemischs aus Atemgas und medizinischem Gas wirksam appliziert werden.

Ferner ist es vorteilhaft, mehr als zwei parallel angeordnete Regulierungsmittel vorzusehen. Es hat sich bei Versuchen herausgestellt, dass es besonders vorteilhaft ist, vier parallel angeordnete Regulierungsmittel vorzusehen, wobei die Regulierungsmittel derart ausgebildet sind, dass mindestens zwei der Regulierungsmittel im geöffneten Zustand eine unterschiedliche Gasmenge durchlassen. Die parallel angeordneten Regulierungsmittel sind vorzugsweise Ventile und werden dann auch als Ventilbank bezeichnet.

Als besonders vorteilhaft hat sich dabei erwiesen, wenn bei den gegebenen Druckverhältnissen das erste Ventil einen Durchfluss von 0,16 Liter pro Minute, das Ventil 2 einen Durchfluss von 1,6 Litern pro Minute sowie die Ventile 3 und 4 jeweils einen Durchfluss von 8 Litern pro Minute bei permanenter Öffnung (gemessen mit medizinischer Luft) haben. Ferner ist es vorteilhaft, wenn Regulierungsmittel genutzt werden, die eine kürzeste realisierbare Öffnungszeit von ≤ 7 Millisekunden, vorzugsweise im Bereich von 4 Millisekunden bis 7 Millisekunden, haben. Die Steuereinheit kann die Ventile einzeln oder in einer beliebigen Kombination öffnen, sodass bei den konkreten Ausführungsbeispielen ein maximaler Durchfluss von 17,76 Liter pro Minute möglich ist.

Besonders vorteilhaft ist es, wenn die Steuereinheit die Öffnung der Regulierungsmittel dahingehend optimiert, dass eine möglichst kurze Öffnungszeit innerhalb eines Atemzuges erreicht wird. Dadurch wird eine reproduzierbare Einmischung des medizinischen Gases in die dem Patienten zugeführte Atemluft erreicht. Ferner wird dadurch ein großer einstellbarer Dosierbereich des an dem Patienten zu applizierenden medizinischen Gases erreicht.

Bei einer Ausführungsform werden bei der Öffnung nur eines Ventils mit einem Durchfluss von 0,16 Litern pro Minute, 7 Millisekunden Öffnungszeit pro Gasimpuls 18,60 Mikroliter des medizinischen Gases appliziert. Durch den erforderlichen Ventilhub und/oder die Ansprechverzögerung werden bei praktischen Versuchen mit diesen Parametern jedoch 13 Mikroliter appliziert. Selbst bei einem Frühgeborenen, das ein Atemzugvolumen von 2 Millilitern (sehr geringe Ventilationsleistung) hat, ist dadurch eine geringe Konzentration von 0,1 ppm je Atemzug bei einer Ausgangskonzentration des medizinischen Gases von 1000 ppm einstellbar. Dadurch kann nach einer höher konzentrierten Applikation des medizinischen Gases diese schrittweise oder kontinuierlich verringert werden, wodurch eine Entwöhnung des Patienten von dem medizinischen Gas bzw. von dessen Wirkstoff einfach möglich ist. Sollte die kleinste pro Atemzug dosierbare Menge immer noch eine pharmakologische Abhängigkeit des Patienten bewirken, kann eine Reduktion der Dosierung des medizinischen Gases dadurch erzielt werden, dass periodisch bei einzelnen Atemzügen die Einleitung des medizinischen Gases ausgesetzt wird. Ferner ist es durch die Verwendung von mehreren parallel geschalteten Regulierungsmittel möglich, bei den derzeit üblichen Applikationskonzentrationen, d. h. Zielkonzentrationen, auch höher konzentrierte Versorgungsgasquellen zu verwenden, wodurch diese Versorgungsgasquellen, insbesondere Versorgungsgasflaschen, in größeren Zeitabständen ausgetauscht werden müssen, wodurch Logistik und Verbrauchskosten gesenkt werden können. Alternativ oder zusätzlich wird durch die Erfindung ein größeres therapeutisches Konzentrationsspektrum klinisch verfügbar.

Wie bereits erwähnt, ist es vorteilhaft, wenn die Regulierungsmittel in geöffnetem Zustand voreinander verschiedene Volumenströme von der Gasquelle zur Patientenzuleitung durchlassen. Bei mehr als zwei Regulierungsmitteln ist es vorteilhaft, wenn zumindest zwei der Regulierungsmittel im geöffneten Zustand unterschiedliche Volumenströme von der Gasquelle zur Patientenzuleitung durchlassen. Dadurch kann eine Konzentration aus einem relativ großen Konzentrationsspektrum auf einfache Art und Weise eingestellt werden.

Besonders vorteilhaft ist es, wenn die Regulierungsmittel jeweils mindestens ein Magnetventil umfassen. Ferner kann zumindest ein Regulierungsmittel eine Drosselblende oder ein anderes Drosselmittel zur Beschränkung des durch das Regulierungsmittel strömenden Volumenstroms vor- und/oder nachgeschaltet werden. Magnetventile sind einerseits kostengünstig und andererseits haben Magnetventile relativ kurze Ansprechzeiten. Die Magnetventile werden insbesondere binär angesteuert, sodass sie in einem ersten Betriebszustand voll geschlossen und in einem zweiten Betriebszustand voll geöffnet sind. Durch die Drosselmittel zur Beschränkung des durch das Regulierungsmittel strömenden Volumenstroms können Regulierungsmittel desselben Typs verwendet werden, insbesondere Magnetventile desselben Typs, wobei der im geöffneten Zustand durch die Regulierungsmittel strömende Volumenstrom durch das Vorsehen unterschiedlicher Strömungswiderstände verschieden ist. Dadurch können auf einfache Art und Weise unterschiedliche Volumenströme durch die Regulierungsmittel hindurch erzeugt werden.

Bei einer vorteilhaften Weiterbildung der Erfindung wird der zweiten Leitung Gas entnommen. Zumindest der Anteil des medizinischen Gases und/oder der Anteil eines Reaktionsproduktes des medizinischen Gases in dem entnommenen Gas wird ermittelt. Dabei kann das Gas der zweiten Leitung über eine Messleitung entnommen und einer Analyseeinheit zur Detektion zumindest des Anteils des medizinischen Gases und/oder des Anteils eines Reaktionsprodukts des medizinischen Gases zugeführt werden. Insbesondere kann die Entnahme und Detektion einmal oder mehrmals während eines Einatemvorgangs, vorzugsweise wiederholt bei jedem Einatemvorgang, durchgeführt werden. Dadurch kann die Konzentration des medizinischen Gases in dem Kreislauf einfach ermittelt, überwacht, begrenzt und/oder geregelt werden. Der Innendurchmesser der Messleitung ist vorzugsweise geringer als der Durchmesser der ersten Leitung, der zweiten Leitung und der Patientenzuleitung.

Ferner ist es vorteilhaft, den ermittelten Anteil des medizinischen Gases als Istwert mit einem Sollwert zu vergleichen und bei einer ermittelten Abweichung des Istwerts von dem voreingestellten Sollwert die bei dem Gasimpuls in die Patientenzuleitung eingebrachte Menge des medizinischen Gases abhängig vom Vergleichsergebnis anzupassen. Vorzugsweise wird der Anteil des medizinischen Gases im Kreislauf auf den voreingestellten Sollwert geregelt. Dadurch kann die dem Patienten zu applizierende Menge des medizinischen Gases auf einfache Art und Weise überwacht, auf einen Sollwert begrenzt und/oder konstant gehalten werden. Insbesondere wird hierdurch eine Kumulation des medizinischen Gases im Kreislauf verhindert. Wird zusätzlich oder alternativ zum Anteil des medizinischen Gases der Anteil eines Reaktionsprodukts des medizinischen Gases analysiert, ist es vorteilhaft, den Anteil eines Oxidationsprodukts des medizinischen Gases zu ermitteln. Wird als medizinisches Gas Stickstoffmonoxid (NO) eingesetzt, kann insbesondere der Anteil des Oxidationsprodukts Stickstoffdioxid (NO₂) ermittelt werden. Der Anteil des ermittelten Stickstoffdioxids kann dann mit einem zulässigen Sollwert verglichen werden. Bei Überschreiten des Sollwerts kann dann die Einleitung des medizinischen Gases in die Patientenzuleitung gestoppt oder das Volumen des eingeleiteten medizinischen Gases reduziert werden. Alternativ oder zusätzlich kann der Frischgas-Eintrag des Anästhesiegerätes entsprechen vom Anwender geregelt werden, damit ein schneller Abtrag es Schadgases Stickstoffdioxid erreicht wird. Bei einer zu hohen Konzentration an Stickstoffdioxid in dem Beatmungsgas kann der Patient geschädigt werden, sodass dies zu vermeiden ist. Der Sollwert ist insbesondere derart voreingestellt, dass beim Zuführen einer entsprechenden Menge an medizinischem Gas dieses vollständig oder zumindest zum Großteil während einer Einatemphase vom Patienten aufgenommen werden kann.

Ferner ist es vorteilhaft, wenn das Anästhesiegerät Informationen über ein Strömungsprofil der Atmung des beatmeten Patienten ermittelt. Die Steuereinheit ermittelt in Abhängigkeit dieses Strömungsprofils den Beginn der Einatemphase oder ermittelt den Zeitpunkt des Beginns einer zukünftigen, vorzugsweise der nächsten, Einatemphase. Durch die Ermittlung des Beginns der Einatemphase aus dem mit Hilfe des Anästhesiegeräts ermittelten Strömungsprofil wird erreicht, dass kein separater Sensor zur Ermittlung des Beginns der Einatemphase verwendet werden muss. Insbesondere muss kein separater zusätzlicher Sensor zur Ermittlung von Druckverhältnissen innerhalb des Verbindungsstücks und/oder innerhalb der Patientenzuleitung verwendet werden. Somit wird ein einfacher kostengünstiger Aufbau erreicht.

Bei einer bevorzugten Ausführungsform der Erfindung wird der zeitliche Beginn mehrerer Einatemphasen ermittelt. Jeweils zu Beginn einer jeden Einatemphase wird genau ein Gasimpuls eingeleitet. Somit wird erreicht, dass zu Beginn jeder Einatemphase die für die optimale Aufnahme notwendige Menge an medizinischem Gas, insbesondere stoßartig, zur Verfügung gestellt wird. Somit kann der Patient mit jedem Atemzug die für seine Therapie optimale Menge an medizinischem Gas aufnehmen.

Aus dem Strömungsprofil werden vorzugsweise zusätzlich zu der Einatemphase mindestens eine Ausatemphase und/oder mindestens ein Atemstillstandphase ermittelt. Weder während der Ausatemphase noch während der Atemstillstandphase wird ein Gasimpuls mit medizinischem Gas in die Patientenzuleitung eingeleitet. Somit wird eine Verschwendung des medizinischen Gases vermieden, da weder während der Atemstillstandphase noch während der Ausatemphase das medizinische Gas von dem Patienten aufgenommen werden kann und somit ungenutzt über die zweite Leitung abgeführt würde.

Bei einer bevorzugten Ausführungsform der Erfindung werden Daten mit Informationen über die Zeitpunkte von mindestens zwei vorangegangenen Einatemphasen ermittelt. In Abhängigkeit dieser Daten wird der zeitliche Beginn der Einatemphase ermittelt. Bei einer besonders bevorzugten Ausführungsform der Erfindung wird der Beginn der Einatemphase als auch die Tiefe des Atemzuges auf Grundlage von mehr als zwei vorangegangenen Einatemphasen festgelegt. Zur Ermittlung des Beginns der Einatemphase wird insbesondere das Steuerungssignal des Anästhesiegeräte in Echtzeit verwendet, um zeitgleich mit der zeitlichen pneumatischen Atemgasregelung dem medizinische Gas dem Patienten totraumminimiert zur Verfügung zu stellen. Alternativ oder zusätzlich kann zur Ermittlung des Beginns der Einatemphase der Abstand zwischen dem Beginn der letzten beiden Einatemphasen ermittelt werden und der Beginn der Einatemphase durch Addition dieses Abstands zu dem Zeitpunkt des Beginns der letzten Einatemphase ermittelt.

Bei einer alternativen Ausführungsform kann der Beginn der Einatemphase auch aus dem Gradienten des Strömungsprofils ermittelt werden. In diesem Fall wird nicht der Beginn einer zukünftigen Einatemphase, sondern der Beginn der aktuellen Einatemphase ermittelt.

Ferner ist es vorteilhaft, wenn das Gasvolumen mindestens einer vorangegangenen Einatemphase ermittelt wird. Die Steuereinheit ermittelt in Abhängigkeit dieses Gasvolumens das Gasvolumen der Einatemphase und legt die Menge an medizinischem Gas, die über den Gasimpuls zu Beginn der Einatemphase eingeleitet wird, in Abhängigkeit des ermittelten Gasvolumens fest. Es ist vorteilhaft, wenn das Gasvolumen mehrerer vorangegangener Einatemphasen ermittelt wird und das Gasvolumen der Einatemphase und somit auch die einzuleitende Menge an medizinischem Gas während der Einatemphase in Abhängigkeit der Gasvolumina der mehreren vorangegangenen Einatemphasen festgelegt werden. Zur Ermittlung des Gasvolumens der Einatemphase kann beispielsweise eine Mustererkennung in vorangegangenen Atemphasen verwendet werden. Alternativ kann das Gasvolumen der Einatemphase durch Bildung eines Mittelwerts der Gasvolumina der vorangegangenen Einatemphasen ermittelt werden.

Die Menge an einzuleitendem medizinischem Gas wird insbesondere proportional zum ermittelten Gasvolumen der Einatemphase festgelegt. Das Gasvolumen der vorangegangenen Einatemphase bzw. die Gasvolumina der vorangegangenen Einatemphasen werden insbesondere aus dem mit Hilfe des Anästhesiegeräts ermittelten Strömungsprofils ermittelt. Somit muss kein zusätzlicher separater Gassensor zur Ermittlung des Gasvolumens vorgesehen werden.

Das Anästhesiegerät setzt dem Atemgas vorzugsweise mindestens ein Anästhesiemittel zum Anästhesieren des an das Anästhesiegerät angeschlossenen Patienten zu. Ferner kann eine Zirkulationseinheit zum Erzeugen mindestens einer Strömung des Gases in dem Kreislauf vorgesehen sein

Als Magnetventile werden vorzugsweise zwischen einer voll geschlossenen und einer voll geöffneten Position umschaltbare Ventile eingesetzt, die binär angesteuert werden.

Die Erfindung kann insbesondere in der Neonatologie zur Behandlung von pulmonaler Hypertomie eines Frühgeborenen mit Stickstoffmonoxid angewendet werden. Auch wird Stickstoffmonoxid appliziert, um Patienten nach Organtransplantationen zu behandeln. Die Erfindung kann jedoch auch zur Applikation anderer gasförmiger Medikamente eingesetzt werden.

Abhängig von der klinischen Anwendung können bis zu 10 % des inspirierten Volumens aus einer Gasquelle zur Bereitstellung gasförmiger Medikamente stammen. Eine solche Gasquelle wird auch als additive Gasquelle bezeichnet, da sie zusätzlich zu einer Atemgasquelle bzw. Sauerstoffquelle bereitgestellt wird. Der im Stand der Technik bestehende Nachteil, dass vom Zeitpunkt der Messung der Strömungsgeschwindigkeit des zur Inspiration des Patienten genutzten Atemgases bis zur mechanischen Einstellung eines zur Einleitung des medizinischen Gases genutzten Regelventils eine Verzögerungszeit ergibt und relativ starke Konzentrationsschwankungen des applizierten medizinischen Gases in der dem Patienten bereitgestellten Beatmungsluft bei dynamischen Flussprofilen auftreten, wird durch die Erfindung vermieden. Ferner ist bei bekannten Regelventilen der Regelbereich des durchgeleiteten medizinischen Gases relativ stark begrenzt. Bei Ventilen, die einen großen Durchfluss des medizinischen Gases ermöglichen, können geringe Durchflussraten nur relativ ungenau eingestellt werden. Bei einer weiteren Ausführungsform der Erfindung kann eine diskontinuierliche Einspeisung durch mehrere Gasimpulse in die Atemluft des die erste Leitung, die zweite Leitung, die Patientenzuleitung umfassenden Patientenkreislaufs des Anästhesiegeräts erfolgen.

Weitere Merkmale und Vorteile der Erfindung ergeben sich aus nachfolgender Beschreibung, die die Erfindung anhand von Ausführungsbeispielen im Zusammenhang mit den beigefügten Figuren näher erläutert.

Es zeigen:
- Figur 1: eine schematische Darstellung einer Vorrichtung zur Applikation mindestens eines medizinischen Gases an einen mit Hilfe eines Beatmungsgeräts beatmeten Patienten gemäß einem ersten Beispiel;
- Figur 2: eine schematische Darstellung von Komponenten eines Appliziergeräts zur Applikation des medizinischen Gases;
- Figur 3: eine schematische Darstellung einer Vorrichtung zur Applikation mindestens eines medizinischen Gases an einen mit Hilfe eines Beatmungsgeräts beatmeten Patienten gemäß einem zweiten Beispiel;
- Figur 4: eine Darstellung des zeitlichen Verlaufs der Atmung des beatmeten Patienten und der Applizierung des medizinischen Gases gemäß dem ersten und zweiten Beispiel;
- Figur 5: eine schematische Darstellung einer Vorrichtung zur Applikation mindestens eines medizinischen Gases an einen mit Hilfe eines Beatmungsgeräts beatmeten Patienten gemäß einem dritten Beispiel;
- Figur 6: eine Darstellung des zeitlichen Verlauf der Atmung eines beatmeten Patienten und der Applizierung des medizinischen Gases gemäß dem dritten Beispiel;
- Figur 7: eine Darstellung des zeitlichen Verlaufs der Atmung eines beatmeten Patienten und der Applizierung des medizinischen Gases gemäß einem vierten Beispiel;
- Figur 8: eine schematische Darstellung einer Vorrichtung zur Applikation mindestens eines medizinischen Gases an einen mit Hilfe eines Beatmungsgeräts beatmeten Patienten gemäß einem fünften Beispiel;
- Figur 9: eine schematische Darstellung einer Vorrichtung zur Applikation mindestens eines medizinischen Gases an einen mit Hilfe eines Beatmungsgeräts beatmeten Patienten gemäß einem sechsten Beispiel;
- Figur 10: eine Darstellung des zeitlichen Verlaufs der Atmung eines beatmeten Patienten und der Applizierung des medizinischen Gases mit Hilfe der Vorrichtung nach Figur 8 oder Figur 9;
- Figur 11: eine schematische Darstellung einer Vorrichtung zur Applikation mindestens eines medizinischen Gases an einen mit Hilfe eines Anästhesiegeräts beatmeten und anästhesierten Patienten gemäß einem ersten Ausführungsbeispiel der Erfindung; und
- Figur 12: eine schematische Darstellung einer Vorrichtung zur Applikation mindestens eines medizinischen Gases an einen mit Hilfe eines Anästhesiegeräts beatmeten und anästhesierten Patienten gemäß einem zweiten Ausführungsbeispiel der Erfindung.

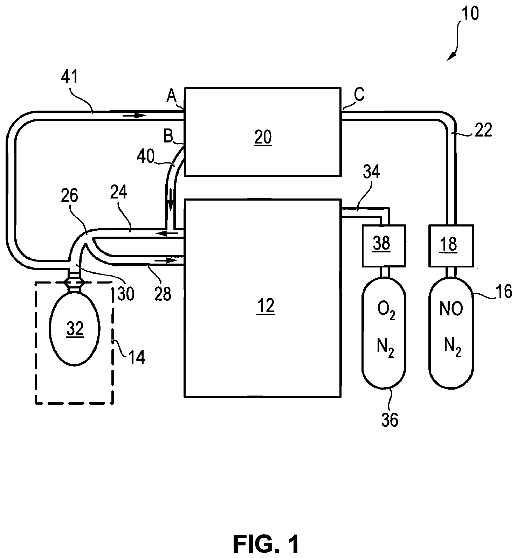

Figur 1 zeigt eine schematische Darstellung einer Vorrichtung 10 zur Applikation mindestens eines medizinischen Gases an einen mit Hilfe eines Beatmungsgeräts 12 beatmeten Patienten 14 gemäß einem ersten Beispiel. In diesem Beispiel wird als medizinisches Gas NO (Stickstoffmonoxid) eingesetzt. Dieses Gas wird in einer Gasflasche 16 als ein N₂-Stickstoff und NO-Stickstoffmonoxid umfassendes Gasgemisch (NO/N₂) bereitgestellt. Mit Hilfe eines Druckreglers 18 wird das Gasgemisch NO/N₂ einer Dosiereinrichtung 20 über einen Verbindungsschlauch 22 mit einem am Druckregler 18 voreingestellten Solldruck am Anschlussstutzen C der Dosiereinrichtung 20 zugeführt. Vom Beatmungsgerät 12 führt eine erste als Atemluftschlauch ausgeführte Leitung 24 zu einem als Y-Stück ausgeführten Verbindungselement 26. Ferner sind eine zweite als Abluftschlauch ausgeführte Leitung 28 und eine Patientenzuleitung 30 mit dem Verbindungselement 26 verbunden. Die Patientenzuleitung 30 ist im vorliegenden Ausführungsbeispiel mit einer den Patienten 14 simulierenden Testlunge in Form eines aufblasbaren Ballons 32 verbunden. Zum Beatmen eines lebenden Patienten 14 ist das zum Patienten 14 führende Ende der Patientenzuleitung 30 mit einer Atemmaske oder einem in die Atemwege des Patienten 14 eingeführten Tubus verbunden. Der Abluftschlauch 28 wird zurück zum Beatmungsgerät 12 geführt, wobei das durch den Abluftschlauch 28 zurückströmende Gasgemisch entweder abgeführt oder im Beatmungsgerät 12 wieder aufbereitet wird. Das Beatmungsgerät 12 ist im vorliegenden Ausführungsbeispiel über einen Verbindungsschlauch 34 mit einer als Gasflasche 36 ausgebildeten Gasquelle verbunden. Die Gasflasche 36 enthält ein Sauerstoff (O₂) und Stickstoff (N₂) umfassendes Gasgemisch (O₂/N₂). Das Gasgemisch O₂/N₂ wird mit Hilfe eines Druckreglers 38 auf einen voreingestellten Sollwert begrenzt und über den Verbindungsschlauch 34 dem Beatmungsgerät 12 zugeführt. Bei anderen Ausführungsbeispielen können Sauerstoff und Stickstoff auch durch getrennte Gasquellen 36 bereitgestellt werden, insbesondere auch über eine zentrale Gasversorgung eines Krankenhauses.

Das Beatmungsgerät 12 erzeugt im Atemluftschlauch 24 einen konstanten Fluss an Atemgas. Diesem konstanten Atemgasfluss wird mit Hilfe der Dosiereinrichtung 20 das zur Behandlung des Patienten bestimmte medizinische Gasgemisch NO/N₂ über die Verbindungsleitung 40 zugeführt. Dazu erzeugt die Dosiereinrichtung einen Gasimpuls abhängig von der Atemfrequenz des Patienten.

Ferner ist eine Messleitung 41 mit der Patientenzuleitung 30 verbunden und führt zumindest ein Teil des in der Patientenzuleitung 30 befindlichen Gasgemischs zum Anschlussstutzen A der Dosiereinrichtung 20. Das der Dosiereinrichtung 20 über den Anschlussstutzen A zugeführte Gasgemisch wird von einer Mess-/Auswerteeinheit 44 der Dosiereinrichtung 20 analysiert.

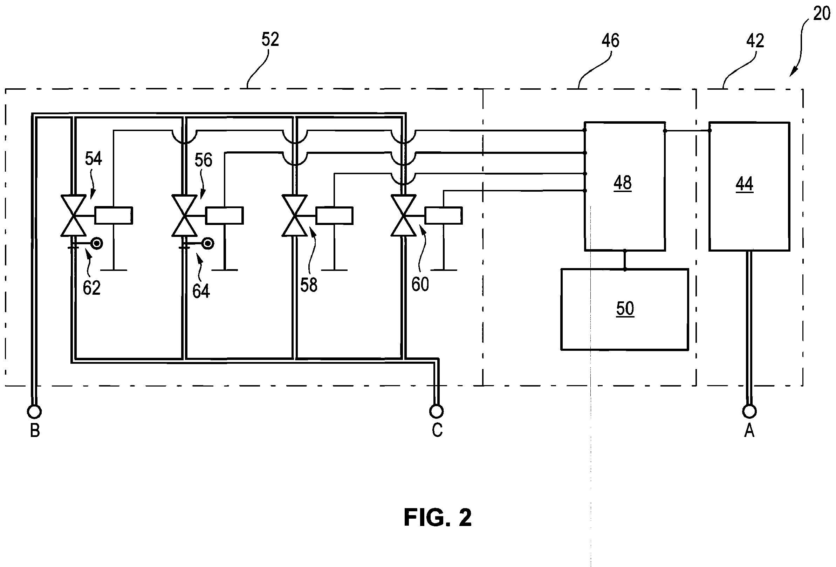

In Figur 2 ist eine schematische Darstellung mit Komponenten der Dosiereinrichtung 20 nach Figur 1 gezeigt. Die Dosiereinrichtung 20 wird auf Grund des im Beispiel als medizinisches Gas verwendeten Stickstoffmonoxids auch als NO-Appliziergerät bezeichnet. Die Dosiereinrichtung 20 hat ein erstes Modul 42 mit einer Mess-/Auswerteeinheit 44, die den Anteil NO im über den Anschlussstutzen A zugeführten Gasgemisch (O₂/N₂/NO) analysiert und einen entsprechenden Messwert an eine im zweiten Modul 46 angeordnete Steuereinheit 48 überträgt. Die Steuereinheit 48 ist mit einer als Mensch-Maschine-Schnittstelle ausgebildeten Bedieneinheit 50 verbunden. Die Bedieneinheit 50 ist vorzugsweise als Touchscreen ausgeführt. Über die Bedieneinheit 50 können Parameter der Dosiereinrichtung 20, insbesondere Sollwerte, eingestellt werden. Ferner können Einstellwerte, Messwerte und Betriebswerte über eine Anzeigeeinheit der Bedieneinheit 50 ausgegeben werden. Die Steuereinheit 48 ist vorzugsweise über eine nicht dargestellte Datenleitung mit einer Steuereinheit des Beatmungsgeräts 12 verbunden. Über diese Datenleitung können relevante Parameter Messwerte und weitere Informationen zwischen der Steuereinheit 48 und der Steuereinheit des Beatmungsgeräts 12 vorzugsweise bidirektional übertragen werden.

Die Dosiereinrichtung 20 hat ein drittes Modul 52, das im vorliegenden Beispiel vier Magnetventile 54 bis 60 umfasst, den jeweils das medizinische Gasgemisch NO/NO₂ über den Anschlussstutzen C zugeführt wird. Den Magnetventilen 54, 56 ist jeweils eine Dosierblende 62, 64 zum Beschränken des Durchflusses durch das jeweilige Magnetventil 54, 56 vorgeschaltet. Die Ausgangsseiten der Magnetventile 54 bis 60 sind mit dem Anschlussstutzen B verbunden, sodass die Magnetventile 54 bis 60 parallel geschaltet sind. Die Steuereinheit 48 der Dosiereinrichtung 20 kann die Magnetventile 54 bis 60 einzeln ansteuern, d. h. einzeln oder in Kombination öffnen. Somit ist es möglich, durch Öffnen eines Ventils 54 bis 60 einen Gasdurchfluss zwischen dem Anschlussstutzen C und dem Anschlussstutzen B zu erreichen und damit medizinisches Gas NO über die Verbindungsleitung 40 in die Atemgasleitung 24 einzuleiten. Die Durchflussmenge zwischen dem Anschlussstutzen C und dem Anschlussstutzen B kann durch das gleichzeitige Öffnen mehrerer Ventile 54 bis 60 erhöht werden. Ferner kann die applizierte Menge, d. h. die in die Atemgasleitung 24 eingeleitete Menge des medizinischen Gases NO durch eine geeignete Wahl der Impulsdauer und/oder durch eine geeignete Wahl der Impulsfrequenz eingestellt werden. Dabei können die von den einzelnen Ventilen 54 bis 60 erzeugten Gasimpulse eine unterschiedliche Impulsdauer bei vorzugsweise gleicher Impulsfrequenz haben. Das dritte Modul mit der parallelen Anordnung mehrerer Ventile 54 bis 60 wird auch als Ventilbank 52 bezeichnet. Die Ventilbank 52 mit den vier Magnetventilen 54 bis 60 ermöglicht einen großen einstellbaren Dosierbereich sowie eine flexible Anpassung der zu applizierenden Gasmenge bei der Verwendung von Gasquellen 16 mit unterschiedlichen Ausgangskonzentrationen des medizinischen Gases. Die Ausgangskonzentration wird vorzugsweise als Parameter über die Bedieneinheit 50 voreingestellt und bei der Berechnung der Impulsdauer und Impulsfrequenz zum Erzeugen der zu applizierenden Menge berücksichtigt.

Im vorliegenden Beispiel hat das Magnetventil 54 einen Durchfluss von 0,16 Liter pro Minute, das Magnetventil 56 einen Durchfluss von 1,6 Liter pro Minute und die Magnetventile 58 und 60 jeweils einen Durchfluss von 8 Litern pro Minute gemessen mit medizinischer Luft.

Mit Hilfe der in Figur 1 gezeigten Anordnung werden bei erwachsenen Patienten Anfangsmaximaldosierungen von 40 ppm und bei Kindern Anfangsmaximaldosierungen von 20 ppm appliziert. Bei Neugeborenen oder Frühgeborenen kann die Anfangsmaximaldosierung geringer sein.

Die Dosierung wird zur Entwöhnung des Patienten schrittweise oder kontinuierlich bis auf 0,5 ppm bei Frühgeborenen bis 0,1 ppm gesenkt. Dabei beträgt die Ausgangskonzentration des medizinischen Gases in der Gasquelle 26 vorzugsweise 1.000 ppm. Alle Dosierungsangaben beziehen sich auf die dem Y-Stück 26 zugeführte Atemluft mit dem eingebrachten medizinischen Gas.

Allgemein ist es durch die Verwendung einer Ventilbank 52 mit mehreren parallel angeordneten Ventilen 54 bis 60 bei derzeit üblichen Applikationsmengen möglich, Gasquellen 16 mit höheren Ausgangskonzentrationen des medizinischen Gases zu verwenden, insbesondere von bis zu 2.000 ppm oder bis zu 4.000 ppm. Gegenüber von Gasquellen mit 1.000 ppm gleicher Gasmenge verlängern sich die Standzeiten bei der Verdopplung der Ausgangskonzentration auf das Doppelte. Alternativ oder zusätzlich bietet die Verwendung der Ventilbank 52 ein größeres therapeutisches Konzentrationsspektrum. Die minimale Öffnungsdauer der Magnetventile 54 bis 60 beträgt im vorliegenden Ausführungsbeispiel 7 Millisekunden. Dadurch kann die in die Atemgasleitung 24 eingeleitete Menge des medizinischen Gases NO in großen Bereichen variiert werden, sodass sich ein großes einstellbares therapeutisches Konzentrationsspektrum ergibt.

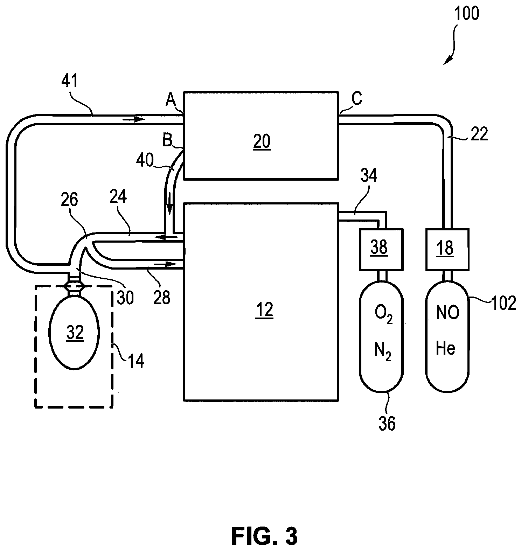

In Figur 3 ist eine schematische Darstellung einer Vorrichtung 100 zur Applikation mindestens eines medizinischen Gases an einen mit Hilfe eines Beatmungsgeräts 12 beatmeten Patienten gemäß einem zweiten Beispiel gezeigt. Die Vorrichtung 100 stimmt strukturell und funktionell mit der Vorrichtung 10 nach Figur 1 überein. Im Unterschied zur Figur 1 wird das medizinische Gas Stickstoffmonoxid (NO) als Gasgemisch bereitgestellt, das Stickstoffmonoxid (NO) und Helium (He) umfasst. Vorzugsweise besteht das Gasgemisch (NO/He) bis auf übliche Verunreinigungen aus Stickstoffmonoxid (NO) und Helium (He). Dieses Gasgemisch (NO/He) wird über eine als Gasflasche ausgebildete Gasquelle 102 bereitgestellt und über den Druckregler 18 und die Verbindungsleitung 22 im Anschlussstutzen C der Dosiereinrichtung 20 zugeführt. Durch das Gasgemisch (NO/He) aus Stickstoffmonoxid (NO) und Helium (He) werden sehr kurze Ansprechzeiten erreicht. Die erzeugten Gasimpulse werden unmittelbar in die Atemgasleitung 24 eingespeist.

Bei Versuchen wurde festgestellt, dass durch die Verwendung eines Gasgemischs (NO/He) aus Stickstoffmonoxid und Helium verglichen mit dem beim ersten Ausführungsbeispiel nach Figur 1 verwendeten Gasgemisch (NO/N₂) aus Stickstoffmonoxid und Stickstoff eine geringere Kompression des Gasgemischs (NO/He) aus Stickstoffmonoxid und Helium erfolgt und somit eine direktere Einleitung des Gasimpulses in die Atemluftzuleitung erfolgt. Dadurch ist auch eine entsprechende impulsartige Partialdruckerhöhung am Patienten 14 messbar, sodass insbesondere die Impulsfrequenz der Gasimpulse vom Patienten 14 spürbar ist. Auch beim Ausführungsbeispiel nach Figur 1 ist eine durch die Gasimpulse bewirkte Partialdruckerhöhung am Patienten 14 messbar. Jedoch bei gleichen Gasimpulsen der Anstieg des Partialdrucks am Patienten 14 sowie der Abfall des Partialdrucks nach einem Gasimpuls bei der Verwendung des Gasgemischs NO/He steiler als bei der Verwendung des Gasgemischs NO/N₂.

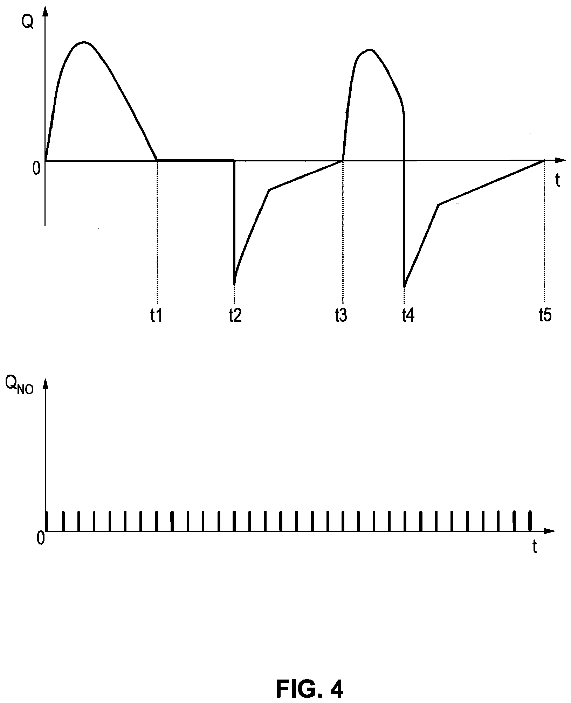

In Figur 4 sind Darstellungen der zeitlichen Verläufe der Atmung des beatmeten Patienten 14 sowie die Applizierung des medizinischen Gases in Form von Gasimpulsen dargestellt. Im oberen Diagramm ist der zeitliche Verlauf der Atmung des Patienten 14 als Volumenstrom Q dargestellt. Im Zeitraum zwischen t0 und t1 erfolgt eine erste Einatemphase des Patienten 14. Im Zeitraum zwischen den Zeitpunkten t1 und t2 erfolgt ein Atemstillstand des Patienten 14. Zwischen dem Zeitpunkt t2 und t3 erfolgt eine erste Ausatemphase des Patienten 14 und zwischen den Zeitpunkten t3 und t4 eine zweite Einatemphase, die gegenüber der ersten Einatemphase kürzer ist. Zwischen den Zeitpunkten t4 und t5 erfolgt eine zweite Ausatemphase.

Im zweiten unteren Diagramm sind die mit Hilfe der Dosiereinrichtung 20 in die Atemluftzuleitung 24 eingeleiteten Gasimpulse als Volumenstrom des relevanten Anteils des medizinischen Gases NO dargestellt. Dabei erfolgt die Zuführung des medizinischen Gases in diesem Beispiel durch Gasimpulse mit einer konstanten Impulsfrequenz und somit unabhängig von der Atemfrequenz des Patienten 14.

Als Magnetventile werden vorzugsweise zwischen einer voll geschlossenen und einer voll geöffneten Position umschaltbare Ventile eingesetzt, die binär angesteuert werden.

Die Erfindung kann insbesondere in der Neonatologie zur Behandlung von pulmonaler Hypertomie eines Frühgeborenen mit Stickstoffmonoxid angewendet werden. Auch wird Stickstoffmonoxid appliziert, um Patienten nach Organtransplantationen zu behandeln. Die in den Beispielen beschriebenen Vorrichtungen 10, 100 können aber auch zur Applikation anderer gasförmiger Medikamente eingesetzt werden.

Ferner ist bekannt, mit Hilfe eines Proportionalventils einem Atemgasstrom gasförmige Medikamente in Abhängigkeit der mit Hilfe eines Strömungsmessers aktuell gemessenen Strömungsgeschwindigkeit des Atemluftstroms beizumischen.

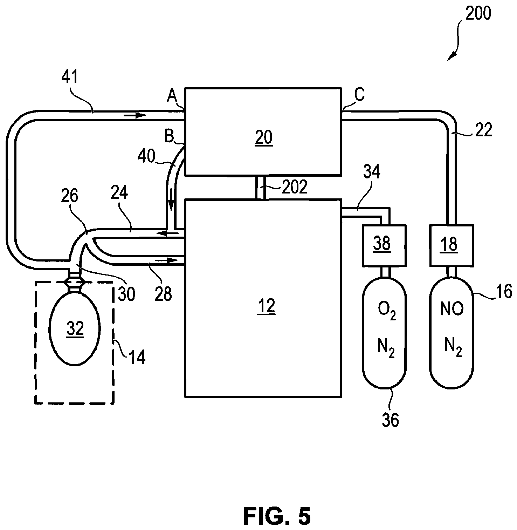

In Figur 5 ist eine schematische Darstellung einer weiteren Vorrichtung 200 zur Applikation mindestens eines medizinischen Gases an einen mit Hilfe eines Beatmungsgeräts 12 beatmeten Patienten 14 gemäß einem dritten Beispiel gezeigt. Im Unterschied zu den Beispielen nach Figur 1 und nach Figur 3 erfolgt die Dosierung des medizinischen Gases NO in das Patientenkreisteil des Beatmungsgeräts 12, d. h. in den Atemluftschlauch 24 proportional zum Atemverlauf des Patienten 14. Im Unterschied zu den Beispielen nach den Figuren 1 und 3 werden beim dritten Beispiel Gasimpulse mit unterschiedlichem Gasvolumen abhängig von der Atemphase und/oder dem Verlauf der Atemphase erzeugt.

Es ist eine Daten- und/oder Signalleitung 202 zwischen dem Beatmungsgerät 12 und der Dosiereinrichtung 20 vorgesehen, über die der Steuereinheit 48 der Dosiereinrichtung 20 Informationen über ein Echtzeitströmungsprofil der Atmung des beatmeten Patienten 14 mit Hilfe von Signalen und/oder Daten übertragen. Zur Datenübertragung kann insbesondere ein echtzeitfähiges Bussystem, wie beispielsweise ein CAN-BUS oder eine serielle Schnittstelle, wie eine USB-Schnittstelle oder RS232-Schnittstelle, unter Verwendung eines echtzeitfähigen Datenübertragungsprotokolls eingesetzt werden.

Die Einleitung des medizinischen Gases in die Atemluftzuleitung 24 erfolgt dann derart, dass während der Atemphasen des Patienten eine höhere Konzentration des medizinischen Gases in der zugeführten Beatmungsluft enthalten ist. Bei einer ersten Ausführungsform des dritten Beispiels werden die Gasimpulse mit einer konstanten Impulsfrequenz abgegeben, wobei die pro Gasimpuls abgegebene Gasmenge während der Einatemphasen größer ist als während Atemstillstandsphasen und während der Ausatemphasen des Patienten 14.

Alternativ oder zusätzlich kann bei weiteren Ausführungsformen die Impulsfrequenz während der Einatemphasen höher sein als während der Ausatemphasen und bei Atemstillstand. Ferner kann bei Atemstillstand des Patienten 14 das Zuführen des medizinischen Gases durch die Dosiereinrichtung 20 unterbrochen sein. Vorteilhaft ist es, mit Hilfe einer durch die Steuereinheit 44 oder einer Steuereinheit des Beatmungsgeräts 12 durchgeführten Optimierung der Gasimpulse und Impulsfrequenz erfolgt dahingehend, dass eine relativ lange Öffnungszeit der aktivierten Ventile 54 bis 60 innerhalb der festgelegten Impulsfrequenz erforderlich ist. Die Impulsfrequenz beträgt vorzugsweise 104 Gasimpulsen pro Minute. Erst dann, wenn der durch die Ventilbank 52 erforderliche Gasdurchfluss des medizinischen Gases größer oder gleich dem maximalen Durchfluss durch ein Ventil 54 bis 60 ist, sodass der Durchfluss durch dieses Ventil 54 bis 60 nicht ausreichen würde, um die erforderliche Menge de4s medizinischen Gases zu applizieren, oder das Ventil 54 bis 60 nicht mehr schließen würde und somit keine Gasimpulse mehr erzeugen würde, wird ein zusätzliches weiteres Ventil 54 bis 60 oder anstatt des ersten Ventils 54 bis 60 ein zweites Ventil 54 bis 60 mit größerem Durchfluss im geöffneten Zustand durch die Steuereinheit 48 angesteuert.

Bei einem vierten Beispiel wird im Unterschied zu dem in Figur 5 gezeigten Beispiel das medizinische Gas NO nicht als Gasgemisch aus Stickstoffmonoxid und Stickstoff (NO/N₂) bereitgestellt, sondern als Gasgemisch aus Stickstoffmonoxid (NO) und Helium (He). Die mit diesem Gasgemisch (NO, He) verbundenen Vorteile sind bereits in Verbindung mit Figur 3 erläutert. Das Erzeugen der Gasimpulse erfolgt bei diesem vierten Beispiel wie für das dritte Beispiel in Verbindung mit Figur 5 beschrieben.

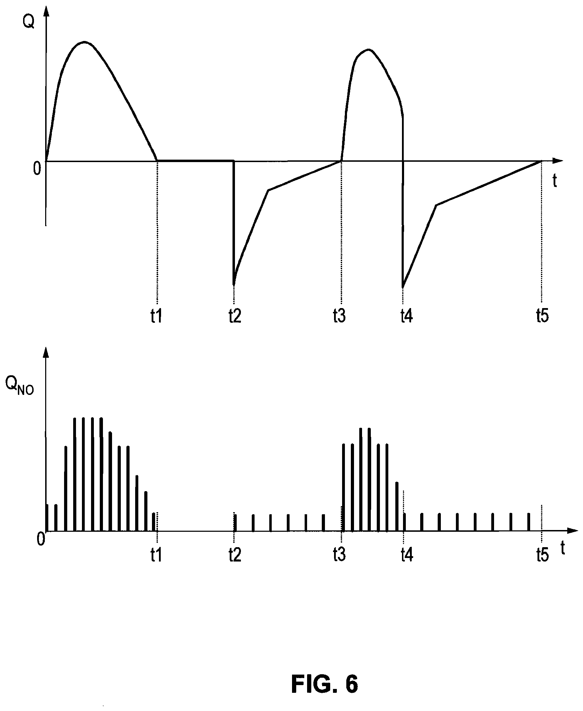

Figur 6 zeigt eine Darstellung des zeitlichen Verlaufs der Atmung des beatmeten Patienten 14 und den zeitlichen Verlauf der Applizierung des medizinischen Gases (NO/N₂)/(NO/He). Dabei ist im oberen Diagramm der Atemluftstrom des beatmeten Patienten 14 dargestellt, ähnlich wie in Figur 4 und im unteren Diagramm sind der zeitliche Verlauf der Gasimpulse dargestellt, durch die das medizinische Gas NO bzw. das Gasgemisch (NO/N₂), (NO/He) in die Atemgaszuleitung 24 eingespeist wird. Dabei ist zu erkennen, dass bei dem gezeigten Beispiel während der Einatemphasen des Patienten der Gasdurchfluss durch die Ventile 54 bis 60 bzw. durch die Ventilbank 52 bei konstanter Impulsbreite durch eine gezielte Auswahl und/oder Kombination unterschiedlicher Ventile 54 bis 60 variiert wird.

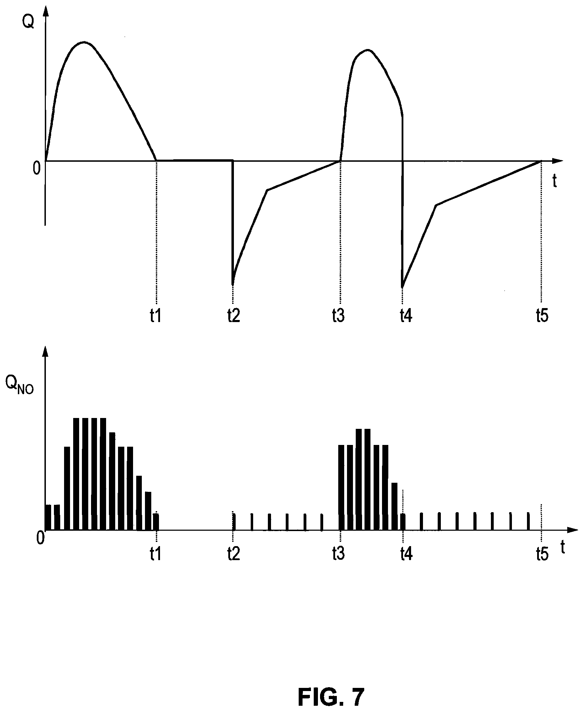

In Figur 7 ist eine Darstellung des zeitlichen Verlaufs der Atmung des beatmeten Patienten 14 und der Applizierung des medizinischen Gases gemäß einem vierten Beispiel gezeigt. Das vierte Beispiel unterscheidet sich von dem in Figur 6 gezeigten Beispiel dadurch, dass während der Einatemphasen Gasimpulse mit einer größeren Impulsbreite als während der Ausatemphasen appliziert werden. Somit wird die applizierte Menge an medizinischem Gas erhöht. Insbesondere kann hierdurch auch bei hohen Strömungsgeschwindigkeiten des Atemgases erreicht werden, dass die Menge an appliziertem medizinischem Gas proportional zur Strömungsgeschwindigkeit ist.

Bei anderen Beispielen kann die bei einem Gasimpuls applizierte Gasmenge dadurch weiter variiert werden, dass die einzelnen Impulsbreiten, mit denen die Ventile 54 bis 60 zum Erzeugen eines Gasimpulses angesteuert werden, verschieden sind, sodass zumindest zwei Ventile 54 bis 60 Gasimpulse unterschiedlicher Impulsbreite abgeben. Dadurch wird ein Gesamtgasimpuls erzeugt, der aus zwei Teilimpulsen unterschiedlicher Impulsbreite erzeugt worden ist. Der Gesamtgasimpuls hat dann einen gestuften Verlauf der in die Atemgaszuleitung 24 eingeleitet wird. Bei einer konkreten Ausführungsform des dritten und vierten Beispiels sind die Impulsfrequenzen während der Einatemphasen doppelt so hoch wie in der Ausatemphase. Beispielsweise kann die Impulsfrequenz während der Einatemphase 208 Gasimpulse pro Minute betragen und während der Ausatemphase 104 Gasimpulse pro Minute. Alternativ kann die Impulsfrequenz während der Einatemphase 104 Gasimpulse pro Minute und während der Ausatemphase 52 Gasimpulse pro Minute betragen. Abhängig vom Anstieg der zu Beginn eines Einatemvorgangs vom Patienten 14 eingeatmeten Gasmenge, d. h. Abhängig vom Durchfluss zu Beginn des Einatemvorgangs und/oder dem zeitlichen Verlauf des Atemgasflusses, kann die Länge eines Einatemzugs des Patienten 14 und/oder der Verlauf des Einatemzugs des Patienten 14 empirisch ermittelt werden und entsprechend dem abgeschätzten Verlauf für jeden Gasimpuls während eines Einatemzugs eine durch diesen Gasimpuls in die Atemgaszuleitung 24 einzuleitende Gasmenge des medizinischen Gases festgelegt werden. Die festgelegte Gasmenge wird dann durch eine geeignete Ansteuerung der Magnetventile 54 bis 60 in die Atemgaszuleitung 24 eingeleitet.

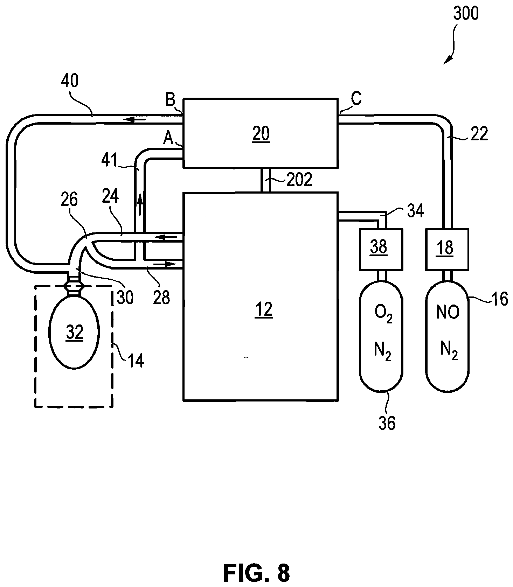

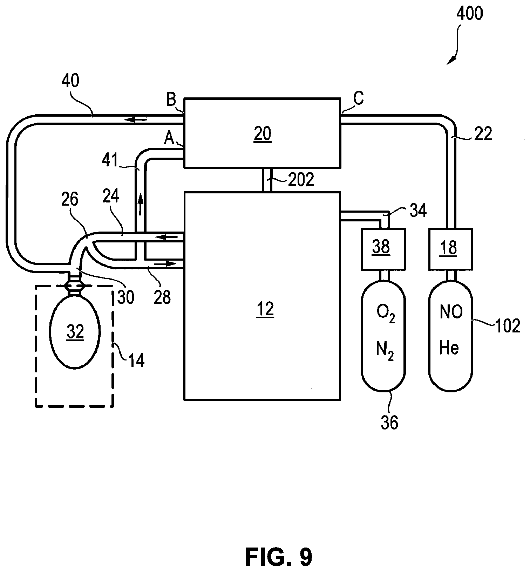

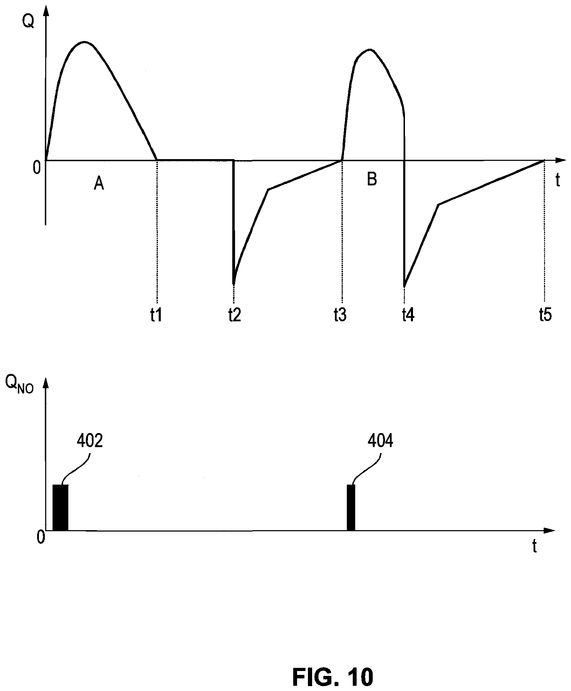

In Figur 8 ist eine schematische Darstellung einer Vorrichtung 300 zur Applikation eines medizinischen Gases NO an einen mit Hilfe eines Beatmungsgeräts 12 beatmeten Patienten 14 gemäß einem fünften Beispiel gezeigt. In Figur 9 ist eine schematische Darstellung einer Vorrichtung 400 zur Applikation mindestens eines medizinischen Gases NO gemäß einem sechsten Beispiel gezeigt. Bei dem fünften Beispiel gemäß Figur 10 wird als Trägergas für das medizinische Gas N2 (Stickstoff) verwendet, wohingegen bei dem sechsten Beispiel nach Figur 9 als Trägergas He (Helium) verwendet wird.

Im Unterschied zu den Beispielen 1 bis 3 wird bei den Beispielen 5 und 6 das medizinische Gas NO über die Gasleitung 40 der Patientenzuleitung 30 direkt und nicht der Atemgasleitung 24 zugeführt. Hierdurch wird erreicht, dass das medizinische Gas dem Patienten 14 möglichst nahe zugeführt wird, sodass es ohne große zeitliche Verzögerung direkt nach dem Zuführen über die Gaszuleitung 40 von dem Patienten 14 aufgenommen werden kann. Dies ist insbesondere bei Behandlung von Säuglingen vorteilhaft, bei denen es sehr wichtig ist, dass das medizinische Gas NO zu den festgelegten Zeitpunkten während der Atmung es Säuglings zugeführt wird.

Ferner unterscheiden sich die Beispiele 5 und 6 von den ersten drei Beispielen dadurch, dass die Messleitung 41 das zu analysierende Gasgemisch nicht aus der Patientenzuleitung 30 sondern aus der Abluftleitung 28 entnimmt. Über die Messleitung 41 wird das Gas der Mess- und Auswerteinheit 44 der Dosiereinrichtung 20 zugeführt.

Wie in Figur 10 dargestellt, erfolgt das Zuführen des medizinischen Gases NO bei der fünften und sechsten Beispielen jeweils stoßartig über einen einzigen Gasimpuls 402, 404 zu Beginn einer jeden Einatemphase. Somit wird gleich zu Beginn einer jeden Einatemphase die von dem Patienten 14 aufzunehmende Menge an medizinischem Gas NO eingeleitet, sodass diese unmittelbar für die Aufnahme durch den Patienten 14 zur Verfügung steht. Somit kann zu Beginn der Einatemphase viel medizinisches Gas NO aufgenommen werden, sodass dieses tief in die Lunge eindringen kann und somit gut von dem Patienten 14 aufgenommen werden kann. Ferner hat dieses stoßartige Zuführen der gesamten Menge an medizinischem Gas NO zu Beginn einer jeden Einatemphase den Vorteil, dass möglichst wenig medizinisches Gas NO ungenutzt während einer Atemstillstandphase und/oder einer Ausatemphase zugeführt wird, während denen es nicht durch den Patienten 14 aufgenommen werden kann.

In Figur 10 ist eine erste Einatemphase zwischen dem Zeitpunkt 0 und dem Zeitpunkt t1 und eine zweite Einatemphase zwischen dem Zeitpunkt t3 und dem Zeitpunkt t4 dargestellt. Wie dem unteren Diagramm zu entnehmen ist, erfolgt am Beginn der ersten Einatemphase, also kurz nach dem Zeitpunkt 0, das Zuführen des medizinischen Gases durch den mit dem Bezugszeichen 402 bezeichneten Gasimpuls. Am Beginn der zweiten Einatemphase erfolgt, kurz nach dem Anfangszeit-Zeitpunkt t3 der Einatemphase, das Zuführen des medizinischen Gases durch den Gasimpuls 404.

Bevorzugt wird mit Hilfe des Beatmungsgeräts 12 ein Strömungsprofil der Atmung des Patienten 14, wie im oberen Diagramm in Figur 10 dargestellt, ermittelt. Aus dem Strömungsprofil wird in Abhängigkeit der Zeitpunkte (0, t3) mindestens zweier unmittelbar vorangegangener Einatemphasen der Zeitpunkt einer zukünftigen oder einer aktuellen Einatemphase ermittelt. Die Steuereinheit 48 steuert die Magnetventile 54 bis 60 derart an, dass zu Beginn der Einatemphase die einzuleitende Gasmenge des medizinischen Gases NO stoßartig über einen Gasimpuls 402, 404 eingeleitet wird. Ferner wird das Gasvolumen mindestens einer vorangegangenen Einatemphase, vorzugsweise mehrerer vorangegangener Einatemphasen, ermittelt. In Abhängigkeit dieses ermittelten Gasvolumens bzw. dieser ermittelten Gasvolumina wird das Gasvolumen der zukünftigen oder aktuellen Einatemphase ermittelt und in Abhängigkeit dessen die in die Patientenzuleitung 30 einzuleitende Menge an medizinischem Gas NO festgelegt. Die einzuleitende Menge an medizinischem Gas NO wird insbesondere derart festgelegt, dass sie umso größer ist, je größer das erwartete Gasvolumen der zukünftigen oder aktuellen Einatemphase ist.

Das Gasvolumen der vorangegangenen Einatemphasen wird insbesondere aus dem Strömungsprofil ermittelt. Hierzu wird insbesondere die von der ermittelten Kurve während der Einatmungsphase und der t-Achse eingeschlossene Fläche ermittelt. Das erwartete Gasvolumen der zukünftigen oder aktuellen Einatemphase wird insbesondere über Mittelwertbildung einer voreingestellten Anzahl vorangegangener Einatemphasen ermittelt. Alternativ kann das erwartete Gasvolumen der zukünftigen oder aktuellen Einatemphase auch mit Hilfe von Mustererkennungen der vorangegangenen Einatemphasen ermittelt werden, durch die beispielsweise periodisch wiederkehrende Schwankungen in der Atmung des Patienten 14 ermittelt werden können.

Wie Figur 10 zu entnehmen ist, ist das Gasvolumen der ersten Einatemphase (0 bis t1) größer als das Gasvolumen der zweiten Einatemphase (t3 bis t4). Entsprechend steuert die Steuereinheit 48 die Magnetventile 54 bis 60 derart an, dass der erste Gasimpuls 402 größer ist als der zweite Gasimpuls 404. Die Größe der Gasimpulse 402, 404, also die Menge an medizinischem Gas, die mit dem jeweiligen Gasimpuls 402, 404 der Patientenzuleitung 30 zugeführt wird, kann durch die Impulsdauer der Gasimpulse 402, 404 sowie durch das Strömungsvolumen des medizinischen Gases während der jeweiligen Gasimpulse 402, 404 eingestellt werden.

Bei einem weiteren alternativen Beispiel ist das medizinische Gas in einem Trägergas, insbesondere Helium, aufgenommen. Hierdurch werden Laufzeitverzögerungen beim Transport des medizinischen Gases durch die Leitungen reduziert, so dass eine genaue Steuerung der Inspirationszeiten möglich ist. Dies ist insbesondere bei der Behandlung von Säuglingen notwendig, da bei ihrer Behandlung Verzögerungen von 100 ms bei den Inspirationszeiten bereits entscheidend über den Erfolg oder Misserfolg der Therapie sein können. Das Beatmungsgerät 12 umfasst einen Sensor zur Berechnung des Gasvolumens eines Atemzuges des Patienten 14 und einen Sensor zur Ermittlung des zeitlichen Beginns eines Atemzuges. Das Beatmungsgerät 12 ist über eine Datenschnittstelle mit der Dosiereinrichtung 20 verbunden, wobei über die Schnittstelle Daten mit Informationen über das Volumen des letzten Atemzuges des Patienten 14 und Daten mit Informationen über die Zeitpunkte der mindestens letzten beiden Atemzüge des Patienten 14 übermittelt werden. Die Steuereinheit 48 ermittelt in Abhängigkeit dieser Daten in Echtzeit den Beginn des nächsten Atemzuges des Patienten 14 und steuert in Abhängigkeit des errechneten Beginns des Atemzuges und zumindest des Gasvolumens des letzten Atemzuges die Magnetventile 54 bis 60 derart an, dass die injizierende Menge an medizinischem Gas zu Beginn des nächsten Atemzuges stoßartig injiziert wird. Unter stoßartiger Injizierung wird insbesondere verstanden, dass das medizinische Gas innerhalb von möglichst kurzer Zeit injiziert wird. Die Steuereinheit 48 öffnet hierzu die Magnetventile 54 bis 60 zu Beginn des Atemzuges möglichst weit.

Das Beatmungsgerät 12 der Vorrichtungen 10, 100, 200, 300 ,400 nach den Figuren 1 bis 10 kann insbesondere Teil eines Anästhesiegeräts zum Beatmen und Anästhesieren des Patienten 14 sein. In diesem Fall wird dem Atemgas mindestens ein Anästhesiemittel zugesetzt.

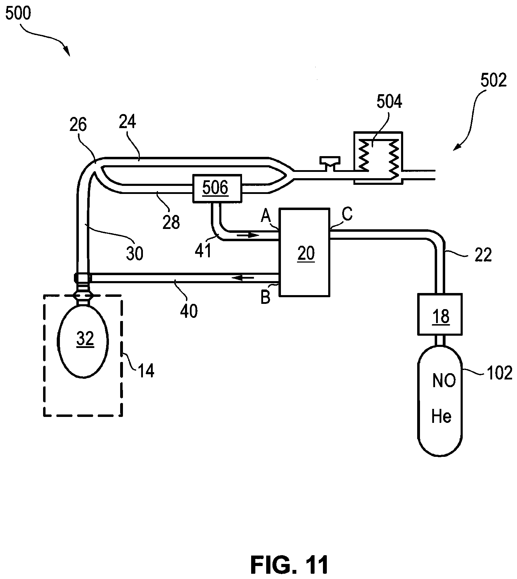

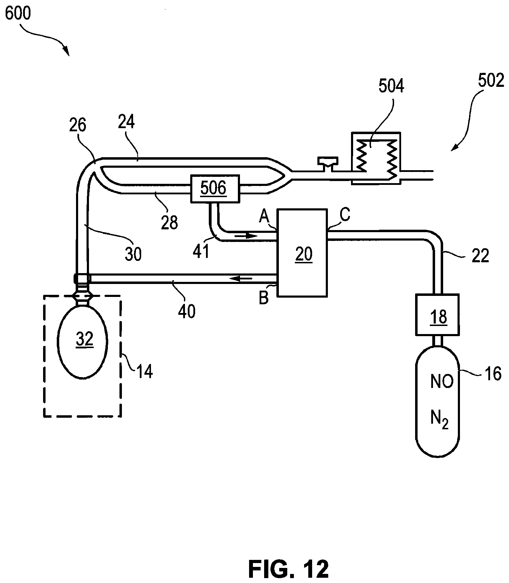

In Figur 11 ist eine schematische Darstellung einer Vorrichtung 500 zur Applikation mindestens eines medizinischen Gases NO an einen mit Hilfe eines Anästhesiegeräts 502 beatmeten und anästhesierten Patienten 14 gemäß einem ersten Ausführungsbeispiel der Erfindung gezeigt. Figur 12 zeigt eine schematische Darstellung einer Vorrichtung 600 zur Applikation mindestens eines medizinischen Gases NO an einen mit Hilfe eines Anästhesiegeräts 502 beatmeten und anästhesierten Patienten 14 gemäß einem zweiten Ausführungsbeispiel der Erfindung. Beim ersten Ausführungsbeispiel wird als Trägergas für das medizinische Gas He (Helium), bei dem zweiten Ausführungsbeispiel N2 (Stickstoff) verwendet. Ansonsten sind die Vorrichtungen 500, 600 analog aufgebaut.

Sowohl beim ersten als auch beim zweiten Ausführungsbeispiel ist der Patient 14 an ein jeweils nur teilweise dargestelltes Anästhesiegerät 502 angeschlossen. Der Patient 14 wird über das Anästhesiegerät, wie bereits zuvor in Zusammenhang mit dem Beatmungsgerät 12 beschrieben, beatmet. Dem Atemgas wird mindestens ein Anästhesiemittel zum Anästhesieren des Patienten 14 zugesetzt. Die Atemgaszuleitung 24 und die Abluftleitung 28 sind an den dem Y-Stück 26 entgegengesetzten Enden miteinander verbunden, so dass ein geschlossener Kreislauf ausgebildet ist und keine Luft, insbesondere kein Anästhesiemittel, in die Umgebungsluft gelangt. Die Vorrichtung 500, 600 umfasst eine Zirkulationseinheit 504 zum Erzeugen mindestens einer Strömung des Gases in dem Kreislauf.

Das medizinische Gas wird vorzugsweise stoßartig über jeweils einen Gasimpuls 402, 404 zu Beginn einer jeden Einatemphase, wie es in Zusammenhang mit Figur 10 beschrieben ist, der Patientenzuleitung 30 zugeführt. Im Unterschied zu dem fünften und sechsten Beispiel wird bei den Ausführungsbeispielen eins und zwei der Erfindung das Strömungsprofil nicht durch ein Beatmungsgerät 12, sondern durch das Anästhesiegerät 502 ermittelt.

In der Abluftleitung 28 ist eine Entnahmeeinheit 506 angeordnet, über die Gas aus dem Kreislauf entnommen und der Messleitung 41 zugeführt werden kann. In dem entnommen Gas wird insbesondere zumindest der Anteil an dem medizinischen Gas ermittelt. Die Steuereinheit 48 regelt über eine entsprechende Ansteuerung der Magnetventile 54 bis 60 den Anteil auf einen voreingestellten Sollwert.

Durch das Stoßartige Zuführen des medizinischen Gases zu Beginn einer jeden Einatemphase in einem Gasimpuls 402, 404 wird erreicht, dass zu Beginn jeder Einatemphase die für die Aufnahme der durch den Patienten 14 aufzunehmenden Menge an medizinischem Gas notwendige Menge an medizinischem Gas zur Verfügung gestellt wird. Somit kann der Patient 14 mit jedem Atemzug die für seine Genesung optimale Menge an medizinischem Gas aufnehmen. Ferner wird hierdurch erreicht, dass bei entsprechender Dosierung der Menge des medizinischen Gases dieses vollständig oder zumindest zum Großteil vom Patienten 14 aufgenommen wird, so dass es zu keiner Kumulation des medizinischen Gases in dem geschlossenen Kreislauf kommt. Somit kann die therapeutisch optimale Menge an medizinischem Gas dem Patienten 14 verabreicht werden und es werden Reaktionen des medizinischen Gases mit anderen Bestandteilen des in dem Kreislauf befindlichen Gasgemisches vermieden oder zumindest reduziert.

Bei der Erfindung ist ein geschlossenes Kreislaufssystems ausgebildet, so dass das vom Patienten 14 ausgeatmete Gasgemisch in dem geschlossenen Kreislaufsystem verbleibt. Somit verbleibt auch das von dem Patienten nicht aufgenommene medizinische Gas in dem Kreislaufsystem. Derartige geschlossene Kreisläufe werden insbesondere bei einer Anästhesie des Patienten 14 verwendet. Während einer Anästhesie ist der Patient 14 mit einem Anästhesiegerät verbunden. Die Steuereinheit 48 ist über eine Schnittstelle mit dem Anästhesiegerät verbunden. Das Anästhesiegerät umfasst mindestens einen Sensor zur Ermittlung des Beginns eines Atemzuges des Patienten 14 und einen Sensor zur Ermittlung der bei diesem Atemzug eingeatmeten Volumenmenge an Gasgemisch. Das Anästhesiegerät übermittelt über die Schnittstelle Daten mit Informationen über den Beginn des Atemzuges und die eingeatmete Volumenmenge an Gasgemisch an die Steuereinheit 48, die in Abhängigkeit dieser Daten die Menge des über die Ventile 54 bis 60 injizierenden medizinischen Gases derart ermittelt, dass so viel medizinisches Gas injiziert wird, dass dieses bei dem Atemzug vollständig oder zumindest nahezu vollständig von dem Patienten 14 aufgenommen wird, so dass es zu keiner Akkumulation des medizinischen Gases in dem Gasgemisch des geschlossenen Kreislaufsystems kommt. Die Steuereinheit 48 steuert die Magnetventile 54 bis 60 insbesondere derart an, dass die zu injizierende Menge an medizinischem Gas innerhalb kurzer Zeit zu Beginn des Atemzuges injiziert wird. Somit wird eine Akkumulation des medizinischen Gases im Gasgemisch vermieden, wodurch beispielsweise Reaktionen mit anderen Substanzen im geschlossenen Kreislaufsystem vermieden werden.

### Bezugszeichenliste

- 10, 100, 200, 300, 400, 500, 600: Vorrichtung
- 16, 36, 102: Gasflaschen
- 12: Beatmungsgerät
- 14: Patient
- 18, 38: Druckregler
- 20: Dosiereinrichtung
- 22, 34: Gaszuleitung
- 24: Atemgasleitung
- 26: Verbindungsstück / Y-Stück
- 28: Abluftleitung
- 30: Patientenzuleitung
- 40: Gaszuleitung
- 41: Messleitung
- 42: erstes Modul
- 44: Mess-/Auswerteeinheit
- 46: zweites Modul
- 48: Steuereinheit
- 50: Bedieneinheit
- 52: drittes Modul / Ventilbank
- 54 bis 60: Magnetventil
- 62, 64: Drosselblende
- 202: Daten- und/oder Signalleitung
- 402, 404: Gasimpuls
- 502: Anästhesiegerät
- 504: Zirkulationseinheit
- 506: Entnahmeeinheit
- A, B, C: Anschlussstutzen
- t1 bis t5: Zeitpunkte

## Patentansprüche

1. Vorrichtung zur Applikation mindestens eines medizinischen Gases an einen mit Hilfe eines Anästhesiegeräts beatmeten und anästhesierten Patienten,
mit einer zumindest Atemgas vom Anästhesiegerät (502) zuführenden ersten Leitung (24),
mit einer zumindest vom Patienten (14) ausgeatmetes Gas abführenden zweiten Leitung (28),
mit einer Patientenzuleitung (30), wobei ein erstes Ende der ersten Leitung (24), ein erstes Ende der zweiten Leitung (28) und ein erstes Ende der Patientenzuleitung (30) über mindestens ein Verbindungsstück (26) miteinander verbunden sind,
mit einem Anästhesiegerät (502), das einen Atemgasfluss erzeugt, und
mit Zuführmitteln zum Zuführen des medizinischen Gases in die Patientenzuleitung (30), mit mindestens zwei parallel angeordneten Regulierungsmittel (54 bis 60), die eine Gasquelle (16, 102) zum Bereitstellen des zu applizierenden medizinischen Gases und die Patientenzuleitung (30) verbinden, wobei jedes Regulierungsmittel (54 bis 60) im geöffneten Zustand eine Verbindung zwischen der Gasquelle (16, 102) und der Patientenzuleitung (30) herstellt, wobei ein zweites Ende der ersten Leitung (24) und ein zweites Ende der zweiten Leitung (28) miteinander verbunden sind, so dass ein geschlossener Kreislauf ausgebildet ist, mit einer Steuereinheit (48), die die Regulierungsmittel (54 bis 60) derart ansteuert, dass mindestens ein Regulierungsmittel (54 bis 60) nacheinander mehrere Gasimpulse des medizinischen Gases in die Patientenzuleitung (30) einleitet, wobei die Steuereinheit (48) den zeitlichen Beginn mindestens einer Einatemphase ermittelt, und die Steuereinheit (48) die Regulierungsmittel derart ansteuert, dass diese am ermittelten Beginn der Einatemphase einen Gasimpuls in die Patientenzuleitung (30) einleiten.

2. Vorrichtung nach Anspruch 1, **dadurch gekennzeichnet, dass** die Steuereinheit (48) abhängig von der in die Patientenzuleitung (30) einzubringenden Menge des medizinischen Gases ein zu öffnendes Regulierungsmittel (54 bis 60) zum Erzeugen des Gasimpulses auswählt oder mehrere zu öffnende Regulierungsmittel (54 bis 60) auswählt und die Impulsdauer abhängig von der bei zwischen einer Zuleitung (22) von der Gasquelle (16, 102) und der Patientenzuleitung (30) vorhandenen Druckdifferenz bei dem ausgewählten Regulierungsmittel (54 bis 60) bzw. bei den ausgewählte Regulierungsmitteln (54 bis 60) zu erwartenden Gasdurchfluss festlegt.

## Claims

1. Device for application of at least one medical gas to a ventilated and anaesthetised patient
with the aid of an anaesthesia machine,
with a first line (24) supplying at least respiratory gas from the anaesthesia machine (502),
with a second line (28) venting at least gas exhaled by the patient (14),
with a patient supply line (30), wherein a first end of the first line (24), a first end of the second line (28) and a first end of the patient supply line (30) are connected with each other via at least one connection piece (26),
with an anaesthesia machine (502) that generates a respiratory gas flow, and
with supply means for supplying the medical gas in the patient supply line (30),
with at least two regulating means (54 to 60) arranged in parallel, having a gas source (16, 102) for providing the medical gas to be applied and connecting the patient supply line (30), wherein each regulating means (54 to 60) provides a connection between the gas source (16, 102) and the patient supply line (30) in the open condition, wherein a second end of the first line (24) and a second end of the second line (28) are connected with each other, so that a closed circuit is formed,
with a control unit (48), which controls the regulating means (54 to 60) in such a way that at least one regulating means (54 to 60) initiates several gas pulses of the medical gas, one after the other, into the patient supply line,
wherein the control unit (48) calculates the start in time of at least one inhalation phase, and the control unit (48) controls the regulating means in such a way that the latter initiates a gas pulse in the patient supply line (30) at the calculated start of the inhalation phase.

2. Device according to claim 1, **characterised in that** the control unit (48) selects a regulating means (54 to 60) to be opened for generating the gas pulse depending on the quantity of medical gas to be supplied via the patient supply line (30), or selects several regulating means (54 to 60) to be opened, and determines the pulse duration depending on the pressure differential existing between a supply line (22) from the gas source (16, 102) and the patient supply line (30) for the selected regulating means (54 to 60) or the gas flow to be expected for the selected regulating means (54 to 60).

## Revendications

1. Dispositif pour administrer au moins un gaz médical à un patient sous anesthésie et sous assistance respiratoire au moyen d'un appareil d'anesthésie,
avec un premier tuyau (24) délivrant au moins un gaz respiratoire à partir de l'appareil d'anesthésie (502),
avec un deuxième tuyau (28) en gaz respiratoire évacuant au moins le gaz exhalé par le patient (14),
avec un tuyau d'alimentation du patient (30) dans lequel une première extrémité du premier tuyau (24), une première extrémité du deuxième tuyau (28) et une première extrémité du tuyau d'alimentation du patient (30) sont reliés entre elles par au moins un adaptateur (26),
avec un appareil d'anesthésie (502) produisant un écoulement de gaz respiratoire et reliant avec des moyens d'alimentation visant à amener le gaz médical dans le tuyau d'alimentation du patient (30),
avec au moins deux moyens de régulation (54 à 60) disposés en parallèle reliant une source de gaz (16, 102) destinée à fournir le gaz médical à administrer et le tuyau d'alimentation du patient (30), dans lequel chaque moyen de régulation (54 à 60) établit une connexion à l'état ouvert entre la source de gaz (16, 102) et le tuyau d'alimentation du patient (30), dans lesquels une seconde extrémité du premier tuyau (24) et une seconde extrémité du second tuyau (28) sont interconnectées, de sorte qu'un circuit fermé est formé, avec une unité de commande (48) commandant le moyen de régulation (54 à 60) de telle sorte qu'au moins un moyen de régulation (54 à 60) introduit successivement une pluralité d'impulsions de gaz du gaz médical dans le tuyau d'alimentation du patient (30),
l'unité de commande (48) déterminant le début temporel d'au moins une phase d'inhalation, et l'unité de commande (48) commandant les moyens de régulation de manière à ce qu'ils introduisent une impulsion de gaz dans le tuyau d'alimentation du patient (30) au début déterminé de la phase d'inhalation.

2. Dispositif selon la revendication 1, **caractérisé en ce que** l'unité de commande (48) sélectionne un moyen de régulation (54 à 60) à ouvrir en fonction de la quantité de gaz médical à introduire dans le tuyau d'alimentation du patient (30) en vue de produire l'impulsion de gaz, ou sélectionne plusieurs moyens de régulation (54 à 60) à ouvrir et détermine la durée d'impulsion en fonction de la différence de pression existant entre un tuyau d'alimentation (22) à partir de la source de gaz (16, 102) et le tuyau d'alimentation du patient (30) avec le moyen de régulation (54 à 60) sélectionné ou les moyens de régulation (54 à 60) sélectionnés, et le débit de gaz attendu.
